# EUROPÄISCHE PATENTSCHRIFT

(11) **EP 1 694 380 B1**
(45) Veröffentlichungstag und Bekanntmachung des Hinweises auf die Patenterteilung: **02.03.2011**
(21) Anmeldenummer: 04820420.0
(22) Anmeldetag: 07.12.2004
(51) Int. Cl.: A61M 1/16

(54) **HÄMODIALYSEGERÄT**
HAEMODIALYSIS DEVICE
APPAREIL D'HEMODIALYSE

(30) Priorität: 09.12.2003 DE 10357835
(43) Veröffentlichungstag der Anmeldung: 30.08.2006
(73) Patentinhaber: Fresenius Medical Care Deutschland GmbH, 61352 Bad Homburg (DE)
(72) Erfinder: WINTER, Josef, 66346 Püttlingen (DE)
(74) Vertreter: Dreyhsig, Jörg
(86) Internationale Anmeldenummer: PCT/EP2004/013898
(87) Internationale Veröffentlichungsnummer: WO 2005/058388

(56) Entgegenhaltungen:
- EP-A- 0 046 126
- EP-A- 0 306 241
- DE-A1- 19 825 568

## Beschreibung

Die Erfindung betrifft ein Hämodialysegerät nach dem Oberbegriff des Anspruchs 1.

Die Hämodialyse stellt eine Nierenersatztherapie dar, bei der das Blut eines Patienten in einem extrakorporalen Kreislauf eine erste Kammer eines durch eine semipermeable Membran in zwei Kammern geteilten Hämodialysators durchfließt. Gleichzeitig durchströmt die zweite Kammer eine als Dialysierflüssigkeit bezeichnete Reinigungsflüssigkeit, die sich bezogen auf die Konzentration der Komponenten, die die semipermeable Membran durchdringen können, im Allgemeinen nicht von der eines Gesunden unterscheidet. Zu entfernende Substanzen wie Harnstoff und Kreatinin sind nicht in der Dialysierflüssigkeit enthalten, wodurch diese aufgrund des Konzentrationsgradienten durch die Membran vom Blut in die Dialysierflüssigkeit übertreten. Die Blutkonzentration anderer Stoffe wie Elektrolyte kann durch die Zusammensetzung der Dialysierflüssigkeit beeinflusst werden. Schließlich kann überschüssige Flüssigkeit durch einen Druckgradienten an der Membran aus dem Blut als Ultrafiltrat entfernt werden.

Eine Hämodialysebehandlung erfordert eine Behandlungszeit von mehreren Stunden, um insbesondere den Kreislauf des Patienten nicht unnötig zu belasten. Die Behandlung ist üblicherweise mindestens dreimal pro Woche durchzuführen, was die Bewegungsfreiheit des Dialysepatienten einschränkt. Die überwiegende Zahl der Behandlungen findet ambulant in dafür vorgesehenen Zentren statt, an denen der Dialysepatient seine eventuellen Reisetätigkeiten ausrichten muss. Dazwischen ergeben sich Zeitperioden von höchsten drei Tagen, nach denen sich der Patient einer erneuten Behandlung unterziehen sollte.

Hämodialysegeräte gehen meist mit einem technischen Aufwand einher, der ein Mitführen eines solchen Gerätes z.B. auf Reisen behindert. Selbst wenn das Gerät keinen Anschluss an eine Wasserversorgung benötigt, da fertige Dialysierflüssigkeit in Beuteln verwendet werden soll, sind die handelsüblichen Geräte nicht für einen Betrieb auf Reisen ohne besondere Transportkapazitäten ausgelegt.

Im Stand der Technik sind Geräte bekannt, die im Gegensatz zu Dialysegeräten, die kontinuierlich frische Dialysierflüssigkeit aus Wasser und Konzentraten bereitstellen, in einem Tank mit konstantem Volumen frische Dialysierflüssigkeit für die gesamte Dialysebehandlung vorhalten. Zur Vermeidung von Problemen mit Verunreinigungen ist dabei vorgeschlagen worden, den als starren Behälter ausgebildeten Tank mit einem flexiblen Beutel auszukleiden, der nach der Behandlung verworfen werden kann.

DE 198 25 568 A1 beschreibt einen solchen Beutel mit einer bestimmten Geometrie sowie die zugehörige Halterung im Sinne des starren Behälters. In einem solchen Gerät wird die verbrauchte Dialysierflüssigkeit so in den Beutel zurückgeleitet, dass das Flüssigkeitsvolumen im Beutel konstant bleibt. Ein Flüssigkeitsentzug wird über eine Ultrafiltrationspumpe erreicht, die dem Dialysierflüssigkeitskreislauf Flüssigkeit entzieht.

EP 0 046 126 B1 beschreibt eine künstliche Niere mit einem ähnlichen Aufbau, wobei ein weiterer Beutel innerhalb des Auskleidungsbeutels vorgesehen sein kann, um eine Durchmischung frischer und verbrauchter Dialysierflüssigkeit zu verhindern. Ein ähnliches System zur Vermeidung der Durchmischung der beiden Flüssigkeiten ist auch Gegenstand der US 4,024,059.

Derartige Geräte sind für den Gebrauch auf Reisen insofern nachteilig, als dass sie im Falle der Auslegung für die Bereitstellung frischer Dialysierflüssigkeit für eine gesamte Dialysebehandlung eine beachtliche Menge an Dialysierflüssigkeit vorhalten können müssen, was mit einem entsprechend unhandlichen Volumen einhergeht. Dies erschwert auch die Bereitstellung vorgefüllter Beutel, wodurch im Stand der Technik durchweg von einer Befüllung erst vor Ort ausgegangen wird. Dies erfordert wiederum einen zusätzlichen Aufwand.

EP 0 046 126 B 1 sieht zur Reduzierung des Volumens des starren Behälters auch vor, die Flüssigkeit in dem starren Behälter periodisch auszuwechseln. Letztendlich beruht aber auch dieses Gerät auf dem Prinzip, dass in dem starren Behälter eine ausgeglichene Flüssigkeitsbilanzierung herbeigeführt wird, was grundsätzlich eine zusätzliche Ultrafiltrationseinheit zur Kontrolle des Wasserentzuges notwendig macht.

Der Erfindung liegt daher die Aufgabe zugrunde, ein gattungsgemäßes medizinisches Gerät derart weiterzubilden, dass es einfach aufgebaut ist und wenige Komponenten erfordert sowie platzsparend und leicht ist und damit eine Behandlung auf Reisen erlaubt, die zumindest die Überbrückung zwischen zwei Klinikaufenthalten und damit eine größere Flexibilität des Patienten erlaubt.

Nach der Lehre der Erfindung wird diese Aufgabe durch ein Hämodialysegerät mit den Merkmalen des Anspruchs 1 gelöst. Ausgestaltungen der Erfindung sind Gegenstand der Unteransprüche.

Die Erfindung macht sich das Verfahren der Rezirkulation der Dialysierflüssigkeit zu Nutze. Danach wird in dem Dialysierflüssigkeitskreislauf mehr Flüssigkeit umgewälzt, als inital an frischer Dialysierflüssigkeit zur Verfügung steht. Unabhängig von der Frage der Durchmischung der Dialysierflüssigkeit kommt es auf diese Weise zur Rezirkulation von verbrauchter Dialysierflüssigkeit, was die Effizienz der Dialysebehandlung aufgrund der sich verkleinernden Konzentrationsgradienten mindert. Auch aus diesem Grund ist es bei moderneren Geräten im Allgemeinen gezielt vermieden worden, dass es überhaupt zu einer Rezirkulation kommt, d.h. der starre Behälter ist ausreichend groß dimensioniert worden. Bei den weit verbreiteten Geräten mit kontinuierlich bereitgestellter Dialysierflüssigkeit wird diese Problematik vollständig vermieden.

Ziel der Erfindung ist es jedoch nicht, ein Hämodialysegerät zur routinemäßigen Behandlung der chronischen Niereninsuffizienz bereitzustellen, sondern die Flexibilität der Personen zu erhöhen. Dies kann schon dadurch erreicht werden, dass eine der üblicherweise drei wöchentlichen Dialysebehandlungen mit dem erfindungsgemäßen Hämodialysegerät durchgeführt werden kann. Der Zeitraum zwischen zwei ambulanten Behandlungen in der Klinik kann sich dann bis auf ca. 5 Tage erhöhen.

Für diesen Fall hat sich die Erfindung die Beobachtung zu Nutze gemacht, dass an ein derartiges Dialysegerät weniger Anforderungen als für die Routinebehandlung gestellt werden können. Erfindungsgemäß wird daher ein Dialysierflüssigkeitsbehälter mit einer Flüssigkeitskammer mit flexiblen Wandungen und mit einem initialen Füllvolumen in den starren Behälter eingelegt, so dass er den starren Behälter nicht, voll ausfüllt und initial ein nicht ausgefülltes Restvolumen verbleibt. Zusätzlich umfasst das Hämodialysegerät eine Steuereinheit, die so ausgeprägt ist, dass sie mit Hilfe der Dialysierflüssigkeitsumwälzeinrichtung während einer Umwälzzeitdauer eine Menge an Dialysierflüssigkeit von und in den Dialysierflüssigkeitsbehälter umwälzt, die die initiale Füllmenge übersteigt.

Die erfindungsgemäße Vorrichtung führt daher gezielt eine Rezirkulation der Dialysierflüssigkeit durch den Dialysierflüssigkeitsbehälter herbei, um kleinere Mengen von Dialysierflüssigkeit besser nutzen zu können. Zwar kommt es hierdurch zur Verminderung des Konzentrationsgradienten an der Dialysemembran, doch kann die Dialysierflüssigkeit durchaus mehrmals den Dialysator durchlaufen, bevor die Effizienz des Dialyseeffektes zu weit reduziert wird. Dies verringert die benötigte Menge an frischer Dialysierflüssigkeit von üblicherweise ca. 80-120 Liter beträchtlich. So hat es sich gezeigt, dass ein Gesamtvolumen von 45 Liter frischer Dialysierflüssigkeit für eine überbrückende Behandlung ausreichend ist. Diese Menge braucht zudem nicht in Form eines einzigen Beutels bereitgestellt zu werden. Vielmehr kann es auf mehrere handelsübliche Beutel mit einem Volumen von ca. 4 - 6 Liter verteilt werden.

Gleichzeitig ermöglicht die erfindungsgemäße Gestaltung eine einfache Kontrolle der Ultrafiltration, ohne eine getrennte Vorrichtung hierfür zu erfordern. Aufgrund des verbleibenden Restvolumens innerhalb des starren Behälters ist sichergestellt, dass innerhalb der Umwälzzeit nicht mehr als dieses Restvolumen dem Patienten entzogen werden kann. Durch eine geeignete Anordnung des Dialysierflüssigkeitskreislaufs relativ zum Blutkreislauf, z.B. durch ein hydrostatisches Gefälle, kann dabei sichergestellt werden, dass sich ein Transmembrandruck von der Blut- zur Dialysierflüssigkeitseite einstellt, der zu einer Erhöhung des Flüssigkeitsvolumen auf der Dialysierflüssigkeitsseite führt, die durch das Restvolumen begrenzt ist.

Das Restvolumen sollte dabei so gewählt werden, dass selbst eine Ausschöpfung des Restvolumens während eines kurzen Anfangszeitraums Komplikationen unwahrscheinlich macht. Dies ist insbesondere bei der Verwendung handelsüblicher Beutel mit einem Volumen von 4 - 6 Liter vorteilhaft realisierbar, weil dann die gesamte Ultrafiltrationsmenge auf die verschiedenen Beutelvolumina verteilt werden kann. In diesem Fall ist die Steuereinheit in einer besonders vorteilhaften Ausführungsform geeignet, über Ventile den Ausfluss eines verbrauchten Beutelvolumens an Dialysierflüssigkeit und den Zufluss eines frischen Beutelvolumens an Dialysierflüssigkeit herbeizuführen.

Neben den für den extrakorporalen Blutkreislauf unumgänglichen Komponenten erfordert das erfindungsgemäße Hämodialysegerät damit nur sehr wenige und platzsparende Komponenten für den Dialysierflüssigkeitskreislauf.

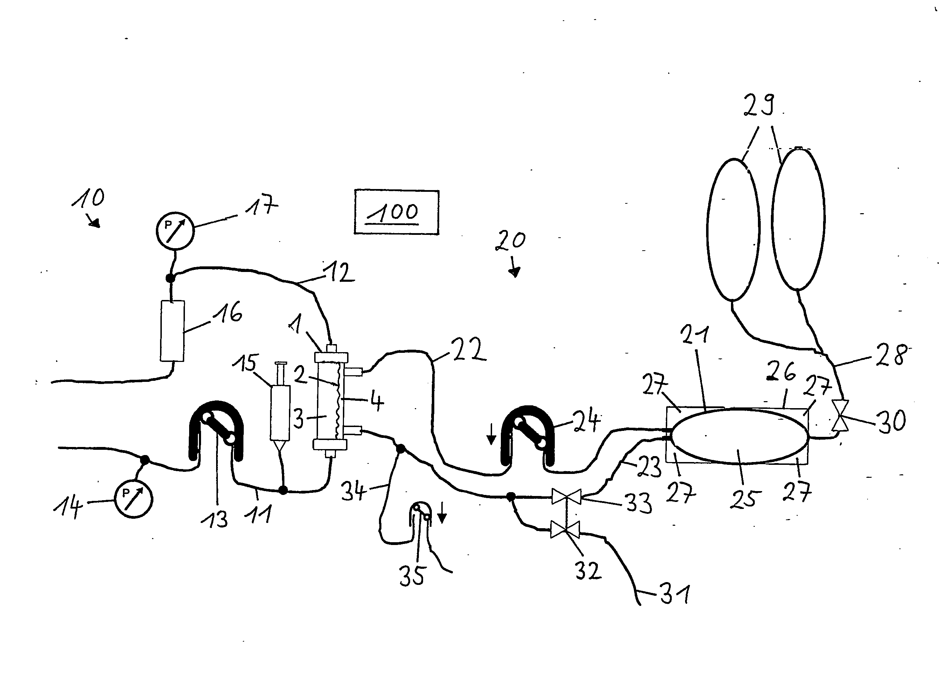

Weitere Einzelheiten und Vorteile der Erfindung werden anhand eines in der einzigen Zeichnung dargestellten Ausführungsbeispiels näher beschrieben. Die dort schematisiert gezeigte Ausführungsform des erfindungsgemäßen Hämodialysegerätes umfasst einen Hämodialysator 1, der durch eine semipermeable Membran 2 in eine erste und zweite Kammer 3 und 4 geteilt ist. Die Membran liegt dabei meist in Form tausender Hohlfasern vor, deren Innenraum die erste Kammer und deren Außenraum die zweite Kammer darstellt.

Die erste Kammer 3 ist Teil eines extrakorporalen Blutkreislaufs 10, dessen Gestaltung dem Fachmann geläufig ist. In der Zeichnung besteht der extrakorporale Blutkreislauf 10 aus einer von einem nicht gezeigten Patienten zur ersten Kammer 3 führenden arteriellen Blutleitung 11 sowie einer von der ersten Kammer 3 zum Patienten führenden venösen Blutleitung 12. In der arteriellen Blutleitung 11 ist eine Blutförderpumpe 13 vorgesehen. Stromaufwärts der Blutförderpumpe 13 ist ein arterieller Blutdrucksensor 14 an die arterielle Blutleitung 11 angeschlossen. Stromabwärts der Blutförderpumpe 13 kann eine Heparinpumpe 15 zum Eintrag eines Antikoagulationsmittels vorgesehen sein. In der venösen Blutleitung 12 ist eine venöse Tropfkammer 16 sowie ein venöser Blutdrucksensor 17 eingebaut.

Die zweite Kammer 4 ist Teil des Dialysierflüssigkeitskreislaufs 20. Dieser besteht im Weiteren aus einem als Beutel ausgeführten Dialysierflüssigkeitsbehälter 21, einer von dem Dialysierflüssigkeitsbehälter 21 zur zweiten Kammer 4 führenden Dialysierflüssigkeitszuführleitung 22 und einer von der zweiten Kammer 4 zum Dialysierflüssigkeitsbehälter 21 führenden Dialysierflüssigkeitsrückführleitung 23. In der Dialysierflüssigkeitszuführleitung 22 ist eine Dialysierflüssigkeitsumwälzeinrichtung 24 eingebaut. Die Dialysierflüssigkeitsumwälzeinrichtung 24 kann auch in der Dialysierflüssigkeitrückführleitung 23 vorgesehen sein, sollten die Transmembrandruckverhältnisse dies erfordern.

Der Beutel 21 umfasst eine aus einer mit flexiblen Wandungen versehene Flüssigkeitskammer 25, die initial mit einer bestimmten Füllmenge an frischer Dialysierflüssigkeit gefüllt ist. Der Beutel 21 ist in einen starren Behälter 26 eingelegt, wobei er diesen bis auf ein Restvolumen 27 ausfüllt. Im einfachsten Fall besteht der Beutel 21 dabei aus einem Schlauchfolienbeutel mit zwei Anschlüssen, die die Flüssigkeitskammer 25 sowohl mit der Dialysierflüssigkeitszuführleitung 22 als auch der Dialysierflüssigekeitrückführleitung 23 verbinden.

Die erfindungsgemäße Gestaltung des Hämodialysegerätes stellt nun auf eine sehr einfache Art sicher, dass dem Patienten während einer Umwälzzeitdauer, in der Dialysierflüssigkeit fortwährend in dem Dialysierflüssigkeitskreislauf 20 umgewälzt wird, es nicht zu einer Überschreitung des Ultrafiltrationsvolumens über einen Grenzwert - dem Restvolumen 27 - und damit auch der mittleren Ultrafiltrationsrate kommen kann. Der Beutel 21 kann sich in diesem Fall maximal auf das Volumen des Inneren des den Beutel vollumgebenden starren Behälters 26 ausdehnen. Eine weitere Ausdehnung wird durch die Wandungen des starren Behälters 26 unmöglich gemacht. Der starre Behälter kann dabei als eine Art Fach ausgebildet sein, dass durch eine Schublade oder Klappe geöffnet und geschlossen werden kann. Die Bedienung ist einfach, da der Benutzer bis auf das Einlegen der Anschlüsse in vorgesehene Öffnungen keine besonderen Handgriffe vornehmen muss. Der starre Behälter 26 kann gleichzeitig geheizte Wandelemente umfassen, um eine bestimmte Temperatur der Dialysierflüssigkeit sicherzustellen. Alternativ kann auch ein Heizelement an der Dialysierflüssigkeitszuführleitung 22 vorgesehen sein.

Die Abmessungen des Volumens des starren Körpers werden vorteilhafterweise auf eine bestimmte Größe handelsüblicher Beutel bezogen, die üblicherweise ein initiales Füllvolumen von 4-6 Liter aufweisen. Das Restvolumen sollte dabei zweckmäßigerweise bei 0,1-0,8 Liter oder bei 0,2-0,8 Liter liegen.

In einer besonders bevorzugten Ausführungsform wird die Umwälzeinrichtung 24 durch eine Steuereinheit 100 genau für eine vorgegebene Umwälzzeitdauer angesteuert, so dass einerseits nicht nur mindestens eine Menge an Dialysierflüssigkeit umgewälzt wird, die die initialle Füllmenge übersteigt, sondern andererseits die Umwälzzeitdauer auch nicht überschritten wird, um eine optimale Ausnutzung der Dialysierflüssigkeit zu gewährleisten. Als vorteilhaft hat sich ein Dialysierflüssigkeitsfluss von mindestens 200 ml/min und eine Umwälzzeitdauer von 30 bis 60 Minuten erwiesen. Zwei bis fünffache Rezirkulationen sind denkbar.

Um ein manuelles Auswechseln des Beutels 21 nach der Umwälzzeitdauer zu vermeiden, ist der Beutel 21 in einer besonders bevorzugten Ausführungsform über eine Leitung 28 mit einer Dialysierflüssigkeitquelle 29 verbunden. Diese besteht im einfachsten Fall aus mehreren parallel verbundenen Dialysierflüssigkeitsbeuteln 29, von denen in der Zeichnung repräsentativ nur zwei gezeigt sind. Die Beutel werden manuell einzeln zugeschaltet und das Ventil 30 regelt den Zulauf in den Beutel 21.

Zusätzlich kann zur Vereinfachung des Wechselprozesses eine Abzweigung in der Dialysierflüssigkeitrückführleitung 23 vorgesehen sein, an die eine Abflussleitung 31 angeschlossen ist, die zu einem nicht näher gezeigten Abfluss führt. In die Abflussleitung 31 ist ein Abflussventil 32 und stromabwärts der Abzweigung in der Dialysierflüssigkeitrückführleitung 23 ein Durchflussventil 33 vorgesehen. Die Ventile 30, 32 und 33 können im einfachsten Fall manuell, im bevorzugten Fall durch die Steuereinheit 100 geschaltet werden.

Während des normalen Dialysebetriebs sind die Ventile 30 und 32 geschlossen, das Durchflussventil 33 geöffnet. Ist die Umwälzzeitdauer erreicht, wird das Ventil 33 geschlossen und das Ventil 32 geöffnet. Die fortlaufende Umwälzeinrichtung 24 pumpt dann den Beutel 21 während einer Ausflusszeit leer, die sich aus dem Volumen des starren Behälters 26 und der Umwälzrate ergibt.

Es ist auch möglich, das Durchflussventil 33 aufstromig der Abzweigung in der Dialysierflüssigkeitsrückführleitung 23 vorzusehen. Zur Entleerung des Beutels 21 braucht dann lediglich die Umwälzeinrichtung 24 angehalten, das Durchflussventil 33 geschlossen und das Abflussventil 32 geöffnet zu werden. Da an den starren Behälter 26 keine exakt luftdichten Anforderungen gestellt werden müssen, kann der Beutel 21 aufgrund eines tiefer gelegenen Abflusses leerlaufen. Zur Verbesserung der Belüftung des starren Behälters 26 können an geeigneten Stellen zusätzliche kleine Belüftungsöffnungen vorgesehen sein, die keine effektive Vergrößerung des Restvolumens 27 mit sich bringen, da sie den flexiblen Wandungen des Beutels 21 ausreichend Widerstand entgegenbringen. Diese Öffnungen können insbesondere abhängig von der Geometrie des starren Behälters 26 und des Beutels 21 an charakteristischen Stellen vorgesehen sein.

Anschließend wird das Abflussventil 32 wieder geschlossen und die Dialysierumwälzeinrichtung 24, falls sie nicht schon angehalten ist, gestoppt. Das Ventil 30 wird geöffnet, so dass der Inhalt eines der über dem Beutel 21 aufgehängten Beutel 29 in den Beutel 21 fließen kann. Zu diesem Zweck können in dem starren Behälter 26 wiederum nicht näher gezeigte Belüftungsöffnungen vorgesehen sein.

Nach der Neufüllung des Beutels 21 mit frischer Dialysierflüssigkeit wird das Ventil 30 wieder geschlossen und das Durchflussventil 33 geöffnet. Mit dem Start der Dialysierflüssigkeitsumwälzeinrichtung 24 wird die Dialysebehandlung für eine neue Umwälzzeitdauer fortgesetzt.

Trotz der vereinfachten Kontrolle der Ultrafiltration während der Behandlung kann zusätzlich eine von der Dialysierflüssigkeitsrückführleitung 23 abzweigende Ultrafiltratleitung 34 vorgesehen sein, in die eine Ultrafiltratpumpe 35 eingebaut ist. Dies kann in solchen Fällen förderlich sein, bei denen das Restvolumen 27 aus Sicherheitgründen knapp bemessen ist und lediglich die Handhabung des Beuteleinlegens in den starren Behälter vereinfacht werden soll. Eine solche Vorrichtung hat dann zumindest den Vorteil, dass keine aufwendigen Wegwerfartikel für den Dialysierflüssigkeitsbehälter 21 und auch keine besondere Sorgfalt beim Einlegen des Beutels 21 notwendig sind.

Das erfindungsgemäße Hämodialysegerät ermöglicht eine einfache Kontrolle der entzogenen Flüssigkeitsmenge, ohne eine eigene Vorrichtung zur Flüssigkeitsentziehung notwendig zu machen. Weiterhin kann das Gerät kompakt und platzsparend gestaltet werden, so dass es sich für eine Benutzung auf Reisen zur Verlängerung der klinikfreien Periode eignet. Im einfachsten Fall ist die dialysatseitige Aktorik auf den Betrieb der Dialysierflüssigkeitsumwälzeinrichtung beschränkt. Der Dialysierflüssigkeitsbehälter kann klein und handlich in Form handelsüblicher Beutel mit einem Volumen von 4-6 Litern ausgeführt sein. Die Dialysierflüssigkeit wird dabei zweckmäßigerweise durch Rezirkulation effizient ausgenutzt. Entweder kann dabei über einen manuellen Beutelwechsel oder eine einfache Verbindung mit Vorratsbeuteln und einem Abfluss über manuell betätigte Ventile eine komplette Behandlung durchgeführt werden. Mit geringem technischen Aufwand kann auch die ohnehin vorhandene Steuereinheit eines solchen Gerätes für eine weitgehend automatisierte Neubefüllung des Dialysierflüssigkeitsbehälters eingesetzt werden.

## Patentansprüche

1. Hämodialysegerät mit einem durch eine semipermeable Membran (2) in zwei Kammern unterteilten Hämodialysator (1), dessen erste Kammer (3) Teil eines extrakorporalen Blutkreislaufs (10) und dessen zweite Kammer (4) Teil eines Dialysierflüssigkeitskreislaufs (20) ist,
wobei der Dialysierflüssigkeitskreislauf (20) einen Dialysierflüssigkeitsbehälter (21) mit einer mit flexiblen Wandungen versehenen Flüssigkeitskammer (25), eine von der Flüssigkeitskammer (25) zur zweiten Kammer (4) führende Dialysierflüssigkeitszuführleitung (22), eine von der zweiten Kammer (4) zu der Flüssigkeitskammer (25) führende Dialysierflüssigkeitsrückführleitung (23) sowie eine Dialysierflüssigkeitsumwälzeinrichtung (24) umfasst,
mit einer Steuereinheit (100) zur Steuerung der Dialysierflüssigkeitsumwälzeinrichtung (24),
mit einem starren Behälter (26) zur Aufnahme des Dialysierflüssigkeitsbehälters (21),
**dadurch gekennzeichnet,**
**dass** der Dialysierflüssigkeitsbehälter (21) in den starren Behälter (26) eingelegt ist und ein initiales Füllvolumen an Dialysierflüssigkeit enthält, so dass er den starren Behälter (26) nicht voll ausfüllt und initial ein nicht ausgefülltes Restvolumen (27) in dem starren Behälter (26) verbleibt,
und **dass** die Steuereinheit (100) so ausgeprägt ist, dass sie mit Hilfe der Dialysierflüssigkeitsumwälzeinrichtung (24) während einer Umwälzzeitdauer eine Menge an Dialysierflüssigkeit umwälzt, die die initiale Füllmenge übersteigt, wobei das Restvolumen innerhalb des starren Behälters sicherstellt, dass innerhalb der Umwälzzeitdauer nicht mehr als dieses Restvolumen dem Patienten trotz Ausdehnung des Dialysierflüssigkeitsbehälters (21) entzogen werden kann.

2. Hämodialysegerät nach Anspruch 1, **dadurch gekennzeichnet, dass** das initiale Füllvolumen 4-6 Liter beträgt.

3. Hämodialysegerät nach Anspruch 1 oder 2, **dadurch gekennzeichnet, dass** das initial nicht ausgefüllte Restvolumen 0,1-0,8 Liter beträgt

4. Hämodialysegerät nach einem der vorhergehenden Ansprüche, **dadurch kennzeichnet, dass** der starre Behälter (26) eine Heizeinrichtung umfasst.

5. Hämodialysegerät nach einem der vorhergehenden Ansprüche, **dadurch gekennzeichnet, dass** die Flüssigkeitskammer (25) mit einer weiteren Leitung (28) mit einer Dialysierflüssigkeitsquelle (29) verbunden ist.

6. Hämodialysegerät nach Anspruch 5, **dadurch gekennzeichnet, dass** die Dialysierflüssigkeitsquelle (29) aus einem oder mehreren Beuteln mit frischer Dialysierflüssigkeit besteht.

7. Hämodialysegerät nach Anspruch 6, **dadurch gekennzeichnet, dass** der oder die Beutel (29) ein Volumen von 4-6 Liter frischer Dialysierflüssigkeit umfassen.

8. Hämodialysegerät nach Anspruch 5, **dadurch gekennzeichnet, dass** in der weiteren Leitung (28) mindestens ein Ventil (30) vorgesehen ist.

9. Hämodialysegerät nach einem der vorhergehenden Ansprüche, dass die umgewälzte Menge an Dialysierflüssigkeit das zwei- bis fünffache der initialen Füllmenge ist.

10. Hämodialysegerät nach einem der vorhergehenden Ansprüche, **dadurch gekennzeichnet, dass** die Dialysierflüssigkeitumwälzeinrichtung (24) als Pumpe, insbesondere als Rollenpumpe, in der Dialysierflüssigkeitszuführleitung (22) ausgebildet ist.

11. Hämodialysegerät nach Anspruch 10, **dadurch gekennzeichnet, dass** flussabwärts der Pumpe (24) von dem Dialysierflüssigkeitskreislauf (20) an einer Abzweigungsstelle eine Abflussleitung (31) abzweigt.

12. Hämodialysegerät nach Anspruch 11, **dadurch gekennzeichnet, dass** in der Abflussleitung (31) ein Abflussventil (32) und flussabwärts der Abzweigungsstelle im Dialysierflüssigkeitskreislauf (20) ein Durchflussventil (33) vorgesehen sind.

13. Hämodialysegerät nach Anspruch 12, **dadurch gekennzeichnet, dass** die Steuereinheit (100) geeignet ist, während der Umwälzzeitdauer das Durchflussventil (33) geöffnet und das Abflussventil (32) geschlossen zu halten, nach der Umwälzzeitdauer das Durchflussventil (33) zu schließen und das Abflussventil (32) zu öffnen und die Pumpe (24) anschließend für eine Entleerungszeitdauer solange weiterzubetreiben, bis die Flüssigkeitskammer (21) vollständig entleert ist.

14. Hämodialysegerät nach einem der vorhergehenden Ansprüche, **dadurch gekennzeichnet, dass** von der Dialysierflüssigkeitsrückführleitung (23) eine Ultrafiltratleitung (34) abzweigt, in der eine mit der Steuereinheit verbundene Ultrafiltratpumpe (35) vorgesehen ist.

## Claims

1. A hemodialysis device comprising a hemodialyzer (1) divided into two chambers by a semipermeable membrane (2), whose first chamber (3) is part of an extracorporeal blood circulator system (10) and whose second chamber (4) is part of a dialysis fluid circulatory system (20),
wherein the dialysis fluid circulatory system (20) comprises a dialysis fluid container (21) with a fluid chamber (25) provided with flexible walls, a dialysis fluid supply line (22) leading from the fluid chamber (25) to the second chamber (4), a dialysis fluid return line (23) leading from the second chamber (4) to the fluid chamber (25) and a dialysis fluid circulating device (24),
comprising a control unit (100) to control the dialysis fluid circulating device (24),
comprising a rigid container (26) to receive the dialysis fluid container (21),
**characterized in that**
the dialysis fluid container (21) contains an initial filling volume of dialysis fluid and is inserted into the rigid container (26), so that said fluid does not completely fill said rigid container (26), and initially there remains an unfilled residual volume (27),
and the control unit (100) is formed such that with the aid of a dialysis fluid circulating device (24), it circulates a quantity of dialysis fluid exceeding the initial filling quantity during a circulation time, such that the residual volume within the rigid container ensures that no more than this residual volume can be withdrawn from the patient within this circulation time despite the expansion of the dialysis fluid container (21).

2. The hemodialysis device according to claim 1, **characterized in that** the initial filling volume is 4-6 liters.

3. The hemodialysis device according to claim 1 or claim 2, **characterized in that** the initially unfilled residual volume is 0.1-0.8 liters.

4. The hemodialysis device according to any one of the preceding claims, **characterized in that** the rigid container (26) comprises a heating device.

5. The hemodialysis device according to any one of the preceding claims, **characterized in that** the fluid chamber (25) is connected to a dialysis fluid source (29) with a further line (28).

6. The hemodialysis device according to claim 5, **characterized in that** the dialysis fluid source (29) consists of one or a plurality of bags containing fresh dialysis fluid.

7. The hemodialysis device according to claim 6, **characterized in that** the bag(s) (29) comprise a volume of 4-6 liters of fresh dialysis fluid.

8. The hemodialysis device according to claim 5, **characterized in that** at least one valve (30) is provided in the further line (28).

9. The hemodialysis device according to any one of the preceding claims, **characterized in that** the circulated quantity of dialysis fluid is two to five times the initial filling quantity.

10. The hemodialysis device according to any one of the preceding claims, **characterized in that** the dialysis fluid circulating device (24) is designed as a pump, in particular as a roller pump, in the dialysis fluid supply line (22).

11. The hemodialysis device according to claim 10, **characterized in that** downstream from the pump (24), a drain line (31) branches off from the dialysis fluid circulatory system (20) at a branching point.

12. The hemodialysis device according to claim 11, **characterized in that** a drain valve (32) is provided in the drain line (31), and a flow valve (33) is provided downstream from the branching point in the dialysis fluid circulatory system (20).

13. The hemodialysis device according to claim 12, **characterized in that** the control unit (100) is suitable for keeping the flow valve (33) open and the drain valve (32) closed during the circulation time, closing the flow valve (33) and opening the drain valve (32) after the circulation time, and then continuing to operate the pump (24) for an emptying time until the fluid chamber (21) is completely emptied.

14. The hemodialysis device according to any one of the preceding claims, **characterized in that** an ultrafiltrate line (34), in which an ultrafiltrate pump (35) connected to the control unit is provided, branches off from the dialysis fluid return line (23).

## Revendications

1. Appareil d'hémodialyse comprenant un hémodialyseur (1) divisé en deux chambres par une membrane semi-perméable (2), dont la première chambre (3) fait partie d'un circuit sanguin (10) extracorporel et dont la deuxième chambre (4) fait partie d'un circuit de dialysat (20),
sachant que le circuit de dialysat (20) comprend un récipient de dialysat (21) avec une chambre de liquide (25) munie de parois flexibles, une ligne d'alimentation en dialysat (22) conduisant de la chambre de liquide (25) à la deuxième chambre (4), une ligne de retour de dialysat (23) conduisant de la deuxième chambre (4) à la chambre de liquide (25) ainsi qu'un dispositif de circulation du dialysat (24),
comprenant une unité de commande (100) pour commander le dispositif de circulation du dialysat (24),
comprenant un récipient rigide (26) pour recevoir le récipient de dialysat (21),
**caractérisé en ce que**
le récipient de dialysat (21) est placé dans le récipient fixe (26) et contient un volume de remplissage initial de dialysat de façon à ce qu'il ne remplisse pas complètement le récipient fixe (26) et qu'il reste au départ un volume restant (27) non rempli dans le récipient fixe (26),
et que l'unité de commande (100) est ainsi conçue que, pendant une durée de circulation, elle fait circuler à l'aide du dispositif de circulation du dialysat (24), une quantité de dialysat qui dépasse la quantité de remplissage initiale, sachant que le volume restant à l'intérieur du récipient fixe garantit que pendant la durée de circulation, on ne puisse pas retirer plus que ce volume restant au patient en dépit de la dilatation du récipient de dialysat (21).

2. Appareil d'hémodialyse selon la revendication 1, **caractérisé en ce que** le volume de remplissage initial est de 4-6 litres.

3. Appareil d'hémodialyse selon la revendication 1 ou 2, **caractérisé en ce que** le volume restant initialement non rempli est de 0,1-0,8 litres.

4. Appareil d'hémodialyse selon l'une des revendications précédentes, **caractérisé en ce que** le récipient fixe (26) comprend un dispositif de chauffage.

5. Appareil d'hémodialyse selon l'une des revendications précédentes, **caractérisé en ce que** la chambre de liquide (25) est reliée à une source de dialysat (29) par une ligne supplémentaire (28).

6. Appareil d'hémodialyse selon la revendication 5, **caractérisé en ce que** la source de dialysat (29) est composée d'un ou plusieurs sachet(s) avec du dialysat neuf.

7. Appareil d'hémodialyse selon la revendication 6, **caractérisé en ce que** le(s) sachet(s) (29) contient/contiennent un volume de 4 à 6 litres de dialysat neuf.

8. Appareil d'hémodialyse selon la revendication 5, **caractérisé en ce qu'**au moins une soupape (30) est prévue dans la ligne supplémentaire (28).

9. Appareil d'hémodialyse selon l'une des revendications précédentes, **caractérisé en ce que** la quantité de dialysat circulée fait deux à cinq fois le volume de remplissage initial.

10. Appareil d'hémodialyse selon l'une des revendications précédentes, **caractérisé en ce que** le dispositif de circulation du dialysat (24) est une pompe, en particulier une pompe à rouleaux, dans la ligne d'alimentation en dialysat (22).

11. Appareil d'hémodialyse selon la revendication 10, **caractérisé en ce qu'**en aval de la pompe (24), une ligne d'écoulement (31) dérive du circuit de dialysat (20) sur un point de dérivation.

12. Appareil d'hémodialyse selon la revendication 11, **caractérisé en ce qu'**une soupape d'écoulement (32) est prévue dans la ligne d'écoulement (31) et une soupape d'écoulement (33) est prévue en aval du point de dérivation dans le circuit de dialysat (20).

13. Appareil d'hémodialyse selon la revendication 12, **caractérisé en ce que** l'unité de commande (100) est appropriée pour maintenir la soupape d'écoulement (33) ouverte et la soupape d'écoulement (32) fermée pendant la durée de circulation, pour fermer la soupape d'écoulement (33) et ouvrir la soupape d'écoulement (32) après la durée de circulation et enfin, pour continuer à faire fonctionner la pompe (24) pendant une durée de vidange jusqu'à ce que la chambre de dialysat (21) soit complètement vidée.

14. Appareil d'hémodialyse selon l'une des revendications précédentes, **caractérisé en ce qu'**une ligne d'ultrafiltrat (34) dérive de la ligne de retour de dialysat (23), dans laquelle ligne d'ultrafiltrat une pompe d'ultrafiltrat (35) reliée à l'unité de commande est prévue.
